(19) Europäisches Patentamt
European Patent Office
Office européen des brevets

(11) Publication number: 0 102 737
A1

# (12) EUROPEAN PATENT APPLICATION

(21) Application number: 83304327.6

(22) Date of filing: 27.07.83

(51) Int. Cl.$^3$: A 61 K 7/08

(30) Priority: 30.07.82 US 403892
22.06.83 US 506922

(43) Date of publication of application:
14.03.84 Bulletin 84/11

(84) Designated Contracting States:
AT BE CH DE FR IT LI LU NL SE

(71) Applicant: THE PROCTER & GAMBLE COMPANY
301 East Sixth Street
Cincinnati Ohio 45202(US)

(72) Inventor: Steuri, Christian
3, St James Court
Fairfield Ohio 45014(US)

(72) Inventor: Mermelstein, Robert
2108 Washington Circle
Cincinnati Ohio 45215(US)

(74) Representative: Brooks, Maxim Courtney et al,
Procter & Gamble (NTC) Limited Whitley Road
Longbenton
Newcastle-upon-Tyne NE12 9TS(GB)

## (54) Shampoo compositions and method of cleaning hair therewith.

(57) Shampoo compositions containing a specific quaternary ammonium compound and a betaine surfactant.

EP 0 102 737 A1

0102737

# SHAMPOO COMPOSITIONS AND METHOD OF CLEANING HAIR THEREWITH

Christian Steuri
Robert Mermelstein

The desire to develop products which simultaneously clean and condition hair has long been present. While the desire has long been present, developing such products has presented innumerable problems. Generally the agents which condition hair best are cationic with one or more long fatty hydrocarbon chains. Hair being negatively charged will allow for the cationic portion to attach to the hair while the long fatty chain(s) provide for ease of combing and hair conditioning.

Cationic materials generally cannot be used with good cleaning anionic surfactants and still deliver good hair condition. This meant that other surfactants such as nonionics, amphoterics and zwitterionics were examined by workers in the field. Many of these efforts are reflected in patents issued in the conditioning shampoo area.

U.S. Patent 3,849,348, November 19, 1974 to Hewitt discloses conditioning shampoos containing betaine, cationic and amine oxide surfactants. U.S. Patent 3,697,452, October 10, 1972 to Olson et al discloses shampoo compositions similar to those in Hewitt. Another patent to Hewitt is U.S. Patent 3,755,559, August 28, 1973 disclosing shampoos containing a tertiary amine oxide, a higher alkyl betaine and a soap. U.S. Patent 3,822,312, July 2, 1974 to Sato discloses shampoos containing a quaternary ammonium salt, a betaine and an additional additive. U.S. Patent 3,990,991, November 9, 1961 to Gerstein discloses shampoos containing amphoteric surfactants and quaternary ammonium compounds. U.S. Patent 4,080,310, March 21, 1978 to Ng et al discloses shampoos containing an amphoteric surfactant and a cationic resin. U.S. Patent 4,132,679, January 2, 1979 to Tsutsumi et al discloses shampoos containing a phosphoric acid ester salt and a betaine. U.S. Patent 4,231,903, November 4, 1980 to Lindemann et al discloses shampoos containing a mixture of an amido betaine and a phosphobetaine. U.S. Patent 4,247,538, January 27, 1981 to Barker discloses a conditioning

shampoo containing a betaine, a polypropoxylated quaternary ammonium chloride surfactant and gum arabic. U.S. Patent 4,294,728, October 13, 1981 to Vanlerberghe et al discloses shampoos containing a cationic, amphoteric or zwitterionic surfactant and a diol. U.S. Patent 4,329,335, May 11, 1981 to Su et al discloses a shampoo composition containing a betaine, an amine oxide and a polymerized quaternary compound. U.S. Patent 4,181,634, January 1, 1980 to Kennedy et al discloses shampoos containing a betaine and a bisquaternary compound.

While the above described references disclose compositions containing components of the type used in the present compositions, they do not teach or suggest totally satisfactory answers to the questions of good cleaning and conditioning. It is believed that good cleaning with quaternary compounds is in part dependent on limiting the reacting of the quaternary with the fatty acids in sebum.

In addition the references fail to teach or suggest combining quaternary compounds of the type disclosed herein with particular betaine surfactants.

It is, therefore, an object of the present invention to provide hair conditioning shampoo compositions which provide good cleaning and conditioning. The good cleaning relates to improved sebum emulsification as well as good lather.

It is a further object of the present invention to provide shampoo compositions containing particular quaternary ammonium compounds and betaine surfactants.

These and other objects will become more apparent from the detailed description which follows.

Unless otherwise indicated, all percentages and ratios herein are by weight.

## DISCLOSURE OF THE INVENTION

The compositions of the present invention comprise from about 0.50% to about 10.0% of selected quaternary ammonium compounds, from about 5% to 70% of an amido betaine and from about 20% to about 94.5% water.

## DETAILED DESCRIPTION OF THE INVENTION

The essential as well as optional components of the present compositions are described in detail below.

### Quaternary Ammonium Compound

The quaternary ammonium compound useful in the present compositions is selected from the group consisting of compounds having the structural formulae:

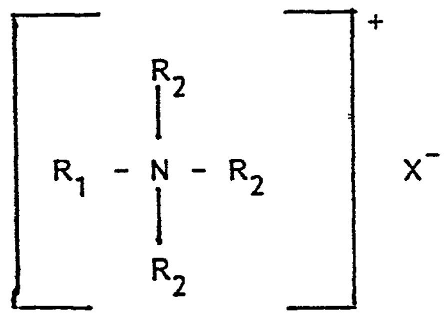

and

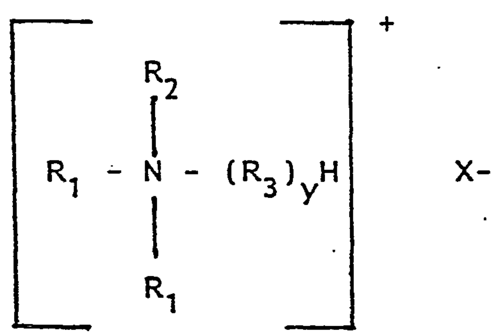

wherein $R_1$ an aliphatic alkyl group containing an average of from about 16 to 22 carbon atoms, most preferably from about 16 to about 18 carbon atoms, the $R_2$ groups are $C_1$ to $C_4$ alkyl or hydroxyalkyl groups, the $R_3$ groups are alkylene oxide groups, preferably propylene oxide, where y is 1 - 4 and X is any compatible anion, particularly one selected from the group consisting of halide, hydroxide, methylsulfate, or acetate anions. Mixtures of these agents are also useful in the present invention. The $R_1$ groups may contain heteroatoms or other linkages, such as hydroxy groups and ester, amide or ether linkages as long as the groups fall within the required carbon atom range.

Preferred quaternary compounds include cetyltrimethyl-ammonium halides (especially chloride). A commercially available

compound of this type is Variquat E228 offered by Sherex Chemical Company. Other preferred quaternary compounds include di(hydrogenatedtallow) 1.5 (propylene oxide) methylammonium methylsulfate. A compound of this type is Varisoft 138 (Sherex).

The level of quaternary compound useful in the present compositions is generally from about 0.50% to about 10.0%, preferably from about 1.0% to about 6.0%.

Surfactant

The essential surfactants used in the compositions of the present invention are higher alkyl amido betaines.

The betaines may be represented by the following structural formula:

$$R_1 - CONH(CH_2)_y - \overset{\overset{\displaystyle R_2}{|}}{\underset{\underset{\displaystyle R_3}{|}}{N^+}} - R_4 - X^-$$

wherein $R_1$ is a long chain alkyl radical having from about 10 to about 18 carbon atoms, $R_2$ and $R_3$ are each alkyl radicals having from about 1 to about 3 carbon atoms, $R_4$ is an alkylene or hydroxy alkylene radical having from about 1 to about 4 carbon atoms, y is an integer from 1 to 4, and X is a carboxylate radical. $R_1$ may be a mixture of long chain alkyl radicals and may contain one or more intermediate linkages or non-functional substituents such as hydroxyl or halogen radicals which do not affect the hydrophobic character of the radical. Examples of betaines useful herein include cocoamidopropyldimethylcarboxymethyl betaine, laurylamidopropyldimethylcarboxymethyl betaine, etc. In many instances the dimethylcarboxymethyl part of the designation is not included.

The amount of surfactant is from about 5% to about 70%, preferably from about 10% to about 25%.

Water

Water is the final essential ingredient of the present invention and forms the remainder of the composition. It is generally

present at a level of from about 20% to about 94.5%, preferably from about 65% to about 80%.

Optional Components

The shampoos herein can contain a variety of non-essential optional components suitable for rendering such compositions more stable and desirable. Such conventional optional ingredients are well known to those skilled in the art, e.g., preservatives such as benzyl alcohol, methyl paraben, propyl paraben and imidazolidinyl urea; other quaternary ammonium compounds which do not meet the above-described formulae such as ditallowdimethylammonium chloride; betaine surfactants such as laurylbetaine in an amount up to about equal to the amount of the amidobetaine; thickeners and vistosity modifiers such as a diethanolamide of a long chain fatty acid (e.g., coconut diethanol amide), sodium chloride, sodium sulfate, methylcellulose, polyvinyl alcohol, and ethyl alcohol; pH adjusting agents such as citric acid, succinic acid, phosphoric acid, sodium hydroxide, sodium carbonate, etc.; suspending agents such as hydrogenated castor oil; opacifiers such as ethylene glycol distearate; perfumes, dyes; and, sequestering agents such as disodium ethylenediamine tetraacetate. Such agents, except for the quaternary ammonium compound and the betaine surfactants, generally are used individually at a level of from about 0.01% to about 5% by weight of the compositions. The pH of the instant compositions is from about 2.5 to about 9, preferably from about 3 to about 6.5.

## METHOD OF MANUFACTURE

The shampoos of the present invention may be made in a variety of methods. A preferred method is set forth in Example I.

## INDUSTRIAL APPLICABILITY

The present compositions are used in a conventional manner for cleaning hair. From about 0.1 g to about 10 g of the composition is applied to wetted hair, worked through the hair and then rinsed out.

The following Examples further describe and demonstrate the preferred embodiments within the scope of the present invention. The Examples are given solely for the purpose of illustration and are not to be construed as limitations of the present invention as many variations thereof are possible without departing from its spirit and scope.

## Example I

The following composition was prepared and is representative of the present invention (all %'s are on a 100% active basis):

| Component | | Wt. % |
|---|---|---|
| Mirataine BB[1] | | 18.0 |
| Variquat E228[2] | | 4.0 |
| Citric Acid | | 4.0 |
| Clindrol Superamide[3] 100CG | | 4.5 |
| Water, perfume, dye and Preservative | q.s. | 100.00% |

1 Lauramidopropyl betaine supplied by Miranol Chemical Company.

2 Cetrimonium chloride supplied by Sherex CHemical Company.

3 Cocamide diethanol amide supplied by Clintwood Chemical Company.

Preparation of Example I

The water, betaine and quaternary ammonium compound are mixed together with agitation and heat. Citric acid is then added. When the temperature reaches about 150°F the cocamide CDA is added and the temperature is increased to the 150°F - 160°F range. The batch is kept at 150°F - 160°F until it is clear. The remaining ingredients are then added.

## Example II

The following is another composition of the present invention:

| Component | | Wt. % |
|---|---|---|
| Aerosol 30[1] | | 18.0 |
| Variquat E228 | | 4.0 |
| Clindrol Superamide 100CG | | 4.0 |
| Water, perfume, dye and Preservative | q.s. | 100.00% |

1 Cocamidopropyl betaine supplied by American Cyanamid.

Example III

The following is another composition of the present invention:

| Component | Wt. % |
|---|---|
| Mirataine BB | 18.0 |
| Adogen 470 DE[1] | 2.0 |
| Variquat E228 | 2.0 |
| Citric Acid | 4.0 |
| Clindrol Superamide 100 CG | 2.0 |
| Water, perfume, dye and preservative q.s. | 100.00% |

1 Di-(hydrogenated tallow) 1.5(propylene oxide)-methylammonium methylsulfate offered by Sherex Chemical Company.

Example IV

The following is another composition of the present invention:

| Component | Wt. % |
|---|---|
| Mirataine BB | 18.0 |
| Variquat E228 | 4.0 |
| Varisoft 138[1] | 2.0 |
| Clindrol Superamide 100 CG | 3.5 |
| Water, perfume dye and preservative q.s. | 100.00 |

1 Di-(hydrogenated tallow) 1.5(propylene oxide)methyl ammonium methyl sulfate offered by Sherex Chemical Company.

Example V

To determine the ability of amido betaine/quaternary ammonium compound compositions to emulsify sebum, the following study was conducted.

Two aqueous solutions were prepared containing 18% cocamidopropyl betaine. To one solution was added 4% cetrimonium chloride and to the other was added 4% stearalkonium chloride.

These solutions were diluted with distilled water to 1/8 of their original concentration. An aliquot of 125 grams of each diluted composition was placed at 100°F for at least two hours.

A sample of each diluted composition was also placed at 100°C until constant weight was obtained.

Artificial sebum, formulated to closely match real sebum, was heated to 100°F and kept at that temperature. This heated sebum in an amount of 5 grams was added as a top layer to each of the 125 grams aliquots which were in separatory funnels.

The separatory funnels were then mounted on a platform capable of rotating at 60 rpm. The funnels were rotated for 960 revolutions after which time the solutions were allowed to settle for 2-1/2 minutes. A 30 g. sample of each solution was placed at 100°C. until constant weight was obtained.

The net change in mass per gram of betaine surfactant was determined. For the composition of this invention:

Fraction of solids in initial dilution = $\frac{\text{solids content found}}{\text{weight of sample}}$

$= \frac{0.48}{15.65} = 0.0307 = Z$

Fraction of solids which is the betaine surfactant

= (wt. of betaine solution)(fraction betaine)/[(wt. of the betaine solution)(fraction betaine + fraction salt impurity) + (wt. of quaternary solution) (fraction quaternary)]

$= \frac{600(0.3096)}{600\ (0.3096 + 0.0466) + (160.1)(0.25)} = 0.7321 = Z'$

Fraction of solids in final composition

$= \frac{\text{solids content found}}{\text{weight of sample}} = \frac{1.63}{27.59} = .0591 = f$

Net change in mass upon agitation = $\delta$

$Z$ (weight of aliquot (W)) + $\delta$ = f (wt. of aliquot + $\delta$)

$\delta = \frac{(Z-f)W}{(f-1)} = \frac{0.0307-0.0591}{0.0591 - 1}\ (124.92) = 3.7716$

Net change in mass per gram of betaine surfactant = y

$y = \frac{\delta}{ZWZ'} = \frac{3.7716}{(0.0307)(124.92)(.7321)} = 1.34$

A similar calculation for the stearalkonium chloride composition showed that there was only 0.08 grams of change in mass per gram of betaine surfactant.

CLAIMS

1. A shampoo composition characterized by:

(A) from 0.5% to 10% of a quaternary ammonium compound selected from

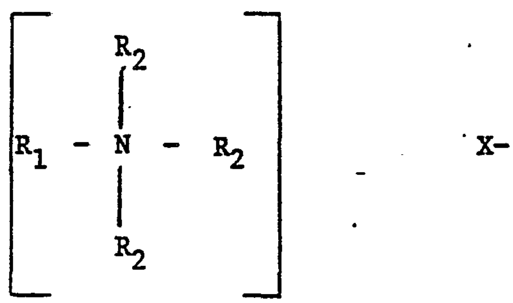

and

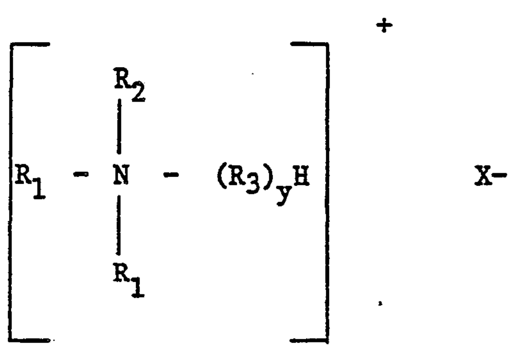

wherein $R_1$ is an alkyl containing an average of from 16 to 22 carbon atoms, $R_2$ is a $C_1$ to $C_4$ alkyl or hydroxy alkyl group, $R_3$ is an alkylene oxide group, y = 1-4 and X is a compatible anion;

(B) from 5% to 70% of a higher alkylamido betaine; and

(C) the remainder water.

2. A shampoo composition according to Claim 1 characterized in that the quaternary ammonium compound is a mono-long chain alkyl tri-methyl ammonium salt or a di-long chain alkylalkylene oxide methyl ammonium salt.

3. A shampoo composition according to Claim 1 or 2 characterized in that the higher alkylamido betaine is a higher alkylamidopropyl betaine.

4. A shampoo composition according to any of Claims 1 to 3 characterized in addition by a diethanol amide of a long chain fatty acid.

5. A shampoo composition according to any of Claims 1 to 4 characterized in that the level of higher alkylamido betaine is from 10% to 25%.

6. A shampoo composition according to any of Claims 1 to 5 characterized in that the level of quaternary ammonium compound is from 1.0% to 6.0%.

7. A shampoo composition according to any of Claims 1 to 6 characterized in that the higher alkylamido betaine is cocoamidopropyl betaine.

8. A shampoo composition according to any of Claims 1 to 7 characterized in that the quaternary ammonium compound is cetyltrimethylammonium chloride or di-(hydrogenated tallow)-1.5 (propylene oxide) methylammonium chloride.

9. A method of cleaning hair comprising:

   a) applying from 0.1g to 10g of a composition according to any of Claims 1 to 8 to hair that has been wetted;

   b) working said composition through said hair; and

   c) rinsing said composition from said hair.

European Patent Office

# EUROPEAN SEARCH REPORT

Application number

EP 83 30 4327

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| X,Y | US-A-3 427 251 (BERGER et al.)<br>* Column 3, line 30 - column 4, line 6 *<br>--- | 1-9 | A 61 K 7/08 |
| Y | CTFA COSMETIC INGREDIENT DICTIONARY, third edition, 1982, pages 46,53, The Cosmetic, Toiletry and Fragrance Association, Inc., Washington, D.C., US<br>* Page 46: "Cetrimonium chloride"; page 53: "Cocamidopropyl betaine" *<br>----- | 2,3,7, 8 | |
| | | | TECHNICAL FIELDS SEARCHED (Int. Cl. 3) |
| | | | A 61 K 7/00<br>C 11 D 1/00 |

The present search report has been drawn up for all claims

| Place of search | Date of completion of the search | Examiner |
|---|---|---|
| THE HAGUE | 14-11-1983 | BENZ K.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document